# EUROPEAN PATENT APPLICATION

(11) **EP 0 950 371 A1**
(43) Date of publication of application: **20.10.1999**
(21) Application number: 97911500.3
(22) Date of filing: 06.11.1997
(51) Int. Cl.: A61B 1/24

(54) **DENTAL MIRROR WITH LIGHTING EQUIPMENT**

(30) Priority: 11.11.1996 JP 1331396 U
(71) Applicant: Tanaka, Hiroshi, Shida-gun, Miyagi 989-41 (JP)
(72) Inventor: Tanaka, Hiroshi, Shida-gun, Miyagi 989-41 (JP)
(74) Representative: Grünecker, Kinkeldey, Stockmair & Schwanhäusser Anwaltssozietät
(86) International application number: JP9704084
(87) International publication number: WO9820789

(57) **Abstract**

A dental mirror with a lighting equipment, which has a light source at the boudary of a mirror portion and a handle portion, and a dental mirror with a lighting equipment, which has a light source around the boundary of a mirror portion and a handle portion. The dental mirror comprises a mirror portion, a boundary, and a handle portion. A small-sized electric lamp is mounted at or around the boundary. A dry cell is inserted into a holder of the handle portion. A switch is mounted to the bottom of the handle portion. With the use of the dental mirror with the lighting equipment, all the portions, such as teeth and gums, of the oral cavity are illuminated from a location close to a portion of the oral cavity by both direct light and reflected light from the mirror to enable inspection with an adequate amount of light.

## Description

### Technical Field

The present invention relates to a dental mirror with lighting equipment having a light source at a boundary portion between a mirror portion and a handle portion, and a dental mirror with a lightning equipment having a light source near to a boundary portion between a mirror portion and the handle portion.

### Background Art

In a conventional dental mirror, in a case that a person uses combined mirrors to inspect his/her teeth, gums or the like, or he/she gets another person to inspect the same, there has been a drawback that it is difficult to inspect the whole inside of his/her mouth due to lack of an equipment for illuminating the inside of mouth or lack of such a functionality as that of a dental illumination equipment.

Japanese Patent Application Publication No. 60-31496, US Patent No 4,212,105, and British Patent No. 1,549,550 are each a dental mirror device comprising a handle body having an lighting member, a mouth-inside mirror and a mouth-outside mirror. The mouth-inside mirror and the mouth-outside mirror are movable. A light-advancing direction is parallel to the handle body.

There are the following drawbacks in the above dental mirror devices.
(1) Since a light source is positioned almost outside a mouth when the dental mirror device is used, lips, cheeks, or teeth become obstacles to light, which prevents illumination in some cases. Also, when inside of a front tooth is inspected, the amount of light is reduced as direct light hardly strike upon the inside or it does not strike upon the same at all.
(2) When outside of a molar tooth is inspected, the amount of light is reduced since direct light strikes upon the outside from a side direction. When it is tried to strike direct light on the outside in order to obtain a sufficient light amount, lips or cheeks must be opened widely.
(3) Experience is required for operation. For this reason, it is difficult for a child to use.
(4) When another person is inspected, the mouth-outside mirror is not used since the combined mirrors are unnecessary. However, as the mouth-outside mirror can not be separated from the dental mirror device, it becomes an obstacle. Also, the dental mirror device is inconvenient to hold and is heavy.

Since there are these drawbacks in the dental mirror device, they have commercially been marketed, but have not widely spread.

### Disclosure of Invention

The present invention has been achieved in order to overcome the above drawbacks.

A dental mirror is provided with lighting equipment which has a light source at or near to a boundary portion between a mirror portion and a handle portion.

A light-advancing direction is set such that the ratio of direct light to reflected light from the mirror is predetermined.

According to the dental mirror with lighting equipment, any portions such as teeth or gum in a mouth are illuminated from a position near to the portion within the mouth by both direct light and reflected light from the mirror, so that an inspection can be performed with a sufficient amount of light.

The direct light and the reflected light from the mirror strike on both the inside of a front tooth and the outside of a molar tooth from the front, so that the light is prevented from be obstructed by lips, cheeks or teeth. A use of the present dental mirror is the same as the conventional dental mirror, and it is very simple to use. It is unnecessary to make the mirror portion movable. The present dental mirror is light, it is easy to handle, and it is handy to carry. When an inspection is performed by a user himself/herself, a wall mirror, a desktop mirror or a hand mirror is used as one of combined mirrors. When an inspection is effected on another person, it is unnecessary to use the combined mirrors.

### Brief Description of Drawings

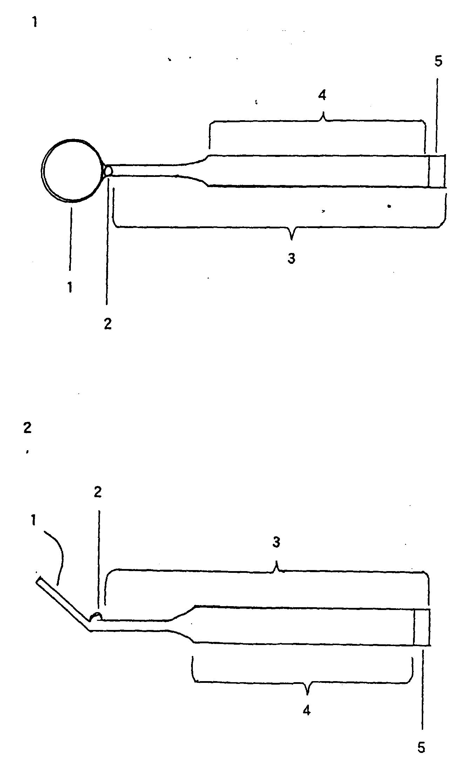

Fig. 1 is a front view, and Fig. 2 is a side view.

Reference character 1 is a mirror portion, 2 is a boundary, 3 is a handle portion, 4 is a holder, and 5 is a switch.

### Best Mode for Carrying Out the Invention

The present invention will be explained with reference to the drawings.

A small-sized light bulb is arranged at or near to a boundary (2) between a mirror portion (1) and a handle portion (3). A dry cell is housed in the holder (4). The switch (5) is provided on a bottom portion of the handle portion (3).

The small light bulb and the dry cell can be housed in the holder (4) so that light is guided therefrom through an optical fiber. In the near future, it is possible to use a light emitting diode. The switch (5) may be arranged on the holder (4).

A battery to be charged may be used instead of the dry cell.

The present invention can be a folding type or a two-dividing type.

It is preferable that both a plane mirror and a concave mirror are prepared as mirrors. The concave mirror serves as a magnifying glass. It is preferred that the mirrors of several sizes are prepared because sizes of mouth are different among persons due to differences in age and person.

A reinforced plastics is preferable to be used for protecting the light bulb, the optical fiber, or like.

The direction degree or projecting degree of the light source is determined or a part of a protecting member is made to be semi-transparent such that the user does not dazzle for the light source to be directly reflected into the eyes of the user. The protecting member is formed in a shape of a convex lens.

It is required to be waterproof. It is also preferable to be chemical resistive against an antiseptic.

## Claims

1. A dental mirror with lighting equipment comprising alight source at a boundary between a mirror portion and a handle portion.

2. A dental mirror with lighting equipment comprising a light source near to a boundary between a mirror portion and a handle portion.
